Europäisches Patentamt

European Patent Office

Office européen des brevets

(11) Publication number: **0 481 657 A2**

# EUROPEAN PATENT APPLICATION

(21) Application number: **91309234.2**

(22) Date of filing: **08.10.91**

(51) Int. Cl.⁵: **C07C 1/207**

(30) Priority: **19.10.90 GB 9022838**

(43) Date of publication of application:
**22.04.92 Bulletin 92/17**

(84) Designated Contracting States:
**AT BE CH DE DK ES FR GB GR IT LI LU NL SE**

(71) Applicant: **THE BRITISH PETROLEUM COMPANY P.L.C.**
**Britannic House, 1 Finsbury Circus**
**London EC2M 7BA(GB)**

(72) Inventor: **Wade, Steven Ronald**
**The British Petroleum Comp. p.l.c., Chertsey Road**
**Sunbury-on-Thames, Middlesex TW16 7LN(GB)**

(74) Representative: **Scott, Susan Margaret et al**
**BP INTERNATIONAL LIMITED Patents & Agreements Division Chertsey Road**
**Sunbury-on-Thames Middlesex TW16 7LN(GB)**

(54) **Process for the conversion of formaldehyde to hydrocarbons.**

(57) A process for the conversion of an aqueous solution of formaldehyde to hydrocarbon products comprising passing the formaldehyde solution over a catalyst bed comprising a metal oxide catalyst component suitable for converting formaldehyde to methanol and/or dimethyl ether and an acid catalyst component suitable for converting the methanol and/or dimethyl ether to hydrocarbons, provided that if said formaldehyde solution comprises methanol, the formaldehyde to methanol molar ratio is greater than 1:1.

EP 0 481 657 A2

The present invention relates to a process for the conversion of formaldehyde to hydrocarbon products.

The preparation of hydrocarbon products suitable for use in gasoline blending is an important commercial concern. In particular, the conversion of methanol to hydrocarbons is known in the prior art. US patent 4645864 discloses a process for the preparation of hydrocarbons including olefins ($C_2$ - $C_5$) in which the feedstock comprises low aliphatic alcohols, carbonyls, ethers or mixtures thereof. A formaldehyde feedstock is, however, not disclosed.

Furthermore, US patent 4374295 discloses a process for converting a methanol feed to light olefins over a zeolite catalyst giving a hydrocarbon product enriched in $C_2$ - $C_4$ olefins. This particular process has a trace of aldehyde in the methanol feedstock.

We have now developed a process for the preparation of hydrocarbons from a relatively dilute formaldehyde feedstock whereby the yield of hydrocarbons is greatly enhanced over the known prior art.

Accordingly, the present invention provides a process for the conversion of an aqueous solution of formaldehyde to hydrocarbon products comprising passing the formaldehyde solution over a catalyst bed comprising a metal oxide catalyst component suitable for converting formaldehyde to methanol and/or dimethyl ether and an acid catalyst component suitable for converting the methanol and/or dimethyl ether to hydrocarbons, provided that, if said formaldehyde solution comprises methanol the formaldehyde to methanol molar ratio is greater than 1:1.

The present invention provides a process in which relatively dilute aqueous solutions of formaldehyde can be converted directly to hydrocarbons. The process utilises a formaldehyde feedstock which may be derived directly from methane or natural gas.

The feedstock of the present invention is a relatively dilute aqueous solution of formaldehyde which may contain methanol. The formaldehyde to methanol ratio is greater than 1:1.

Throughout this specification and claims, it should be understood that the term formaldehyde solution includes solutions of polymers of formaldehyde such as paraformaldehyde. Preferably, the feedstock is an aqueous solution of formaldehyde (HCHO) derived from the partial oxidation of methane or methanol. A method for the partial oxidation of methane and methanol to provide formaldehyde is typically disclosed in Journal of Catalysis, 120, 231-255 (1989) by M A Vest et al.

Optionally, the process of the present invention may comprise at least one co-feed. Suitable co-feeds may be selected from carbon dioxide, carbon monoxide, hydrogen or an inert diluent eg nitrogen.

The catalyst bed used in the present process comprises a metal oxide component and an acid catalyst component.

The metal oxide may suitably be a solid base and/or an amphoteric oxide predominantly basic in character, and may suitably be selected from oxides of lithium, magnesium, calcium, strontium, barium, gallium, zinc, cadmium, tin, titanium, zirconium, hafnium, thorium, and any one of the lanthanides. Equally suitable are combinations of at least two of the aforementioned metals.

Optionally, the metal oxide component may be impregnated with an additional metal if so desired. The additional metal may be selected from copper, platinum and palladium and may be found in a concentration of suitably 0 to 10% wt, preferably 0.01 to 5% wt, especially 0.05 to 1% wt of the metal oxide component.

The acid catalyst may be selected from a class of known zeolite structures as outlined in the "Atlas of Zeolite Structure Types" by Meier W M and Olsen D H (1987) distributed by Polycrystal Book Service, Pittsburgh USA. A code consisting of three capital letters has been adopted for each known structure type following the recommendations by IUPAC on zeolite nomenclature ("Chemical Nomenclature and Formulation of Compositions of Synthetic and Natural Zeolites", IUPAC, yellow booklet, 1978). Example of such structure types are TON, MEL and MFI. TON-type structures are also known as theta-1, which is disclosed in our European Patent No 57049, Nu-10 which is disclosed in European Patent No 065400 and ZSM-22 which is disclosed in Canadian Patent No 1202941. MFI-type structures are also known as ZSM-5 which may, for example, be prepared as described in EP 002899 and EP 002900. MEL-type structures are also known as ZSM-11 which is disclosed in GB 1339501. Also possible are ZSM-23, ZSM-35 and ZSM-48.

These zeolites are usually produced from a silica source, an alumina source, an alkali metal hydroxide and a nitrogen containing base such as ammonia or an alkanolamine such as diethanolamine. Zeolites prepared in this manner are described in our published European Patent Applications 002899 and 002900. The hydrogen form is suitably produced by calcination to remove the nitrogenous base, followed by exchange with an ammonium salt and further calcination.

The zeolites may be used in the present process in their as synthesised form or in the hydrogen form. Equally suitable is a metal exchanged form such as gallium exchanged zeolite and microporous gallo and boro silicates eg GaMEL.

The zeolite may suitably be activated prior to use in the present process. This may suitably be achieved by heating in a flow of air or inert gas such as nitrogen.

Equally suitable are acid clays and heteropolyoxometallates which, as well as the aforementioned zeolites, are distinguished by their ability to convert methanol to hydrocarbons. Examples of such heteropolyoxometallates are disclosed in a paper by Hayashi, H and Moffat, J B, Journal of Catalysis, 77, 476-484 (1982).

The process of the present invention may be suitably carried out at a temperature of from 200-600°C, preferably 350-500°C and under a pressure suitably of from 0.1-100 bar absolute, preferably 0.8 - 50 bar absolute.

The process may be carried out at a gas hourly space velocity suitably of from 200-20000 h$^{-1}$ GHSV, preferably 100-10000 h$^{-1}$ GHSV. It is understood for the purpose of the present process that gas hourly space velocity is defined as the volume of feed gas at STP fed per hour divided by the total volume of the catalyst bed.

The catalysts of the present invention may be suitably arranged in separate, discrete regions of the catalyst bed. Equally suitably, the catalyst bed may comprise a mixed two-component catalyst. Preferably, a mixed catalyst bed is utilised in the present process.

In the absence of an active catalyst or in the presence of a zeolite catalyst alone, formaldehyde is converted to mainly methanol and dimethylether with a selectivity up to approximately 35% C-mol. Surprisingly, the mixed catalyst bed of the present invention gives a mixture of aliphatic and aromatic hydrocarbons with selectivities exceeding 50%. Even more surprising is that hydrocarbon selectivities exceed those possible by a simple combination of formaldehyde disproportionation to methanol/carbon oxides combined with the conversion of methanol to hydrocarbons.

The reaction may be carried out in a variety of reactors such as fixed beds, fluidised beds and riser reactors.

The product of the process is predominantly hydrocarbons comprising $C_2$-$C_{10}$ olefins, alkanes and aromatics. By-products comprise carbon monoxide, carbon dioxide and methane. The use of the binary catalyst provides a process wherein the yield of hydrocarbons is at least 50%.

The invention will now be described in detail with reference to the following examples:

A: Preparation of the zeolite catalyst (H-ZSM-5)

A sample of the base zeolite, referred to as Laporte Zeolite RD119, was purchased from Laporte and treated in the following manner. The zeolite was calcined at 560°C for 24 hours prior to stirring with 1M nitric acid (10 ml acid per gram of zeolite) for a further four hours. The resulting mixture was filtered and the product washed with deionised water (10ml/g). The acid treatment was repeated a second time and the resulting product dried at 70°C for 16 hours. The zeolite was then bound with Ludox AS40 (Trade Mark) colloidal silica (1:1 by weight) and dried to give a solid containing 71.4% zeolite/wt. The bound material was ground and pelleted prior to steam treatment (500°C for 8 hours in a 74% steam/26% nitrogen stream). The material was further washed with 1M nitric acid under flow conditions (10 times volume per weight over 2 hours) and deionised water prior to drying at 70°C. The resulting catalyst had an aluminium content of 2.3%/wt.

B: Preparation of the Mg-ZSM-5 Catalyst

The hydrogen form of the ZSM-5 catalyst, prepared as outlined above, was exchanged with a 0.1M solution of magnesium nitrate (10 x vol/wt) for 30 minutes to provide the magnesium form.

C: Preparation of H-GaMEL

Ludox AS40 colloidal silica (26.7g), sodium hydroxide (1.4g), gallium nitrate (2.45g), tetra butyl ammonium hydroxide solution (40% /wt, 23.1g) and water (46.3g) were combined to give a zeolite precursor gel which was heated at autogeneous pressure at 170°C for 71 hours. The resulting raw material was washed with distilled water, calcined in air (500°C for 20 hours) and exchanged with 1M ammonium chloride solution (3 exchanges, 4 x vol wt, stirred for 2 hours). The resulting ammonium form contained 5.2%/wt gallium and less than 0.01% sodium. A further calcination (500°C/2 hours) converted the ammonium form to the acid form.

D: Preparation of the Binary Catalysts

The metal oxides were used as supplied and pelleted (10 tons/inch) prior to crushing and meshing to 150-350 mm. A 10ml catalyst charge comprising 5 ml of metal oxide and 5 ml of acid catalyst component was used. The binary catalyst was tested as a graduated bed, arranged such that the concentration of the metal oxide component was approximately 100% at the leading edge of the bed, and decreasing linearly to approximately 0% at the exit of the catalyst bed. The acid catalyst component was arranged on a reciprocal arrangement, the concentration being approximately 0% at the leading edge and increasing linearly to approximately 100% at the exit.

Example 1. MgO/H-ZSM5 Catalyst

A formaldehyde feedstock (20.6% mol formaldehyde, 5.7% methanol, 52.2% water and 21.5% nitrogen) was passed over MgO/H-ZSM-5 catalyst prepared according to step D above at 425°C, atmospheric pressure and 3060 h$^{-1}$ GHSV. Product analysis showed 98.1% formaldehyde conversion to a product comprising olefins/alkanes with 55.1% $C_2$- $C_{10}$ selectivity and aromatics with 15.10% selectivity. Selectivity was calculated on a carbon mole basis.

Example 2. MgO/H-ZSM-5 Catalyst

A feedstock as disclosed in Example 1 was passed over a MgO/H-ZSM-5 catalyst at 425°C, atmospheric pressure and 1640 h$^{-1}$ GHSV. Product analysis showed 100% formaldehyde conversion to a product comprising olefins/alkanes with 52.1% $C_2$ - $C_{10}$ selectivity and aromatics with 19.8% selectivity.

Example 3. MO/H-GaMEL

A feedstock as disclosed in Example 1 was passed over a MgO/H-GaMEL catalyst at 425°C, atmospheric pressure and 2470 h$^{-1}$ GHSV. Product analysis showed 100% formaldehyde conversion to a product comprising olefins/alkanes with 39.7% $C_2$ - $C_{10}$ selectivity and aromatics with 18.3% selectivity.

Comparative Example 1 SiO$_2$ Catalyst

Commercial silica chips were crushed in a hammer mill and graded. Repeated extractions with 11M hydrochloric acid and disodium EDTA were carried out to remove any iron contaminants.

A feedstock as disclosed in Example 1 was passed over the silica at 450°C, atmospheric pressure and 2720 h$^{-1}$ GHSV. Product analysis showed 18.5% formaldehyde conversion to a product comprising essentially methanol, carbon monoxide and carbon dioxide. Hydrocarbons were not detected in the product.

Comparative Example 2. H-ZSM-5 Catalyst

A feedstock as disclosed in Example 1 was passed over an H-ZSM-5 catalyst at 425°C, atmospheric pressure and 3000 h$^{-1}$ GHSV. Product analysis showed 78.5% formaldehyde conversion to a product with 20.0% methanol selectivity, 14.5% DME selectivity, 51.3% carbon monoxide selectivity and 3.4% carbon dioxide selectivity. Hydrocarbon selectivity was 5.7% C-mol.

Comparative Example 3. Mg ZSM-5 Catalyst

A feedstock as disclosed in Example 1 was passed over magnesium exchanged H-ZSM-5 catalyst. Product analysis showed 89.2% formaldehyde conversion and 43.2% methanol/dimethyl ether (DME) selectivity. Selectivity to hydrocarbons was 4.3%.

**Claims**

1. A process for the conversion of an aqueous solution of formaldehyde to hydrocarbon products comprising passing the formaldehyde solution over a catalyst bed comprising a metal oxide catalyst component suitable for converting formaldehyde to methanol and/or dimethyl ether and an acid catalyst component suitable for converting the methanol and/or dimethyl ether to hydrocarbons, provided that, if said formaldehyde solution comprises methanol the formaldehyde to methanol molar ratio is greater than 1:1.

2. A process according to claim 1 wherein the metal oxide component is selected from oxides of lithium, magnesium, calcium strontium, barium, gallium, zinc, cadmium, tin, titanium, zirconium, hafnium, thorium, members of the lanthanide series and combinations thereof.

3. A process according to either claim 1 or claim 2 wherein the metal oxide component comprises a second metal selected from copper, platinum or palladium.

4. A process according to any one of the preceding claims wherein the acid catalyst component is a zeolite.

5. A process according to claim 4 wherein the acid catalyst component is selected from MFI, MEL and TON type structures.

6. A process according to any one of claims 1 to 3 wherein the acid catalyst component is selected from an acid clay or a heteropolyacid.

7. A process according to any one of the preceding claims carried out in the temperature range of from 200-600°C.

8. A process according to any one of the preceding claims carried out in the pressure range of from 0.8-50 bar absolute.

9. A process according to any one of the preceding claims wherein a co-feed is selected from carbon dioxide, carbon monoxide, hydrogen and an inert diluent.

10. A process according to any one of the preceding claims wherein the feedstock is obtained directly from the oxidation of methane or methanol.